(19) **Europäisches Patentamt / European Patent Office / Office européen des brevets**

(11) **EP 2 023 257 A2**

(12) # EUROPEAN PATENT APPLICATION

(43) Date of publication:
**11.02.2009 Bulletin 2009/07**

(51) Int Cl.:
**G06F 19/00** [(2006.01)]

(21) Application number: **08013312.7**

(22) Date of filing: **24.07.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **25.07.2007 ES 200702072**

(71) Applicant: **Bap Health Outcomes Research, S.L.**
**33600 Mieres Asturias (ES)**

(72) Inventors:
• **Rebollo Alvarez, Pablo**
**33600 Mieres (Asturias) (ES)**
• **Carcia Cueto, Eduardo**
**33600 Mieres (Asturias) (ES)**

• **Cuervo Uria, Jesus**
**33600 Mieres (Asturias) (ES)**
• **Chanca Zardain, Pilar**
**33600 Mieres (Asturias) (ES)**
• **Martinez Castejon, Ignacio**
**33600 Mieres (Asturias) (ES)**
• **Alonso Caballero, Jordi**
**33600 Mieres (Asturias) (ES)**
• **Ferrer Fores, Montserrat**
**33600 Mieres (Asturias) (ES)**
• **Muniz Fernandez, José**
**33600 Mieres (Asturias) (ES)**

(74) Representative: **Isern-Jara, Nuria**
**Avda. Diagonal, 463 Bis 2°**
**08036 Barcelona (ES)**

(54) **Procedure to evaluate the quality of life related to the health of a person**

(57)    Procedure to evaluate the quality of life related to the health of a person from the data introduced by said person in a computer terminal connected to a data processing central computer. The procedure presents a first question to the subject via the terminal, and it evaluates the response of the subject according to calculated parameters that form part of the system so as to assign a mark to the subject. Subsequently the system identifies the following question from amongst those that make up part of the total base initiating a new process until it stops the presentation of the questions according to its working rules. The procedure includes means for the obtaining of responses form the subject being evaluated; means to generate the evaluation parameter associated to the response of the subject; means to choose the next question to be put to the subject and repeat the process; means to stop the presentation of questions; and means for the presentation of the resultant evaluation of the process for the subject being evaluated.

FIGURE **2**

EP 2 023 257 A2

**Description**

DESCRIPTIVE MEMORANDUM

AIM OF THE INVENTION

The present invention refers to a system to evaluate the quality of life related to the health of a person, and more specifically to an adaptive system of self-assessment of the quality of life related to the health that is based on calculations with methodology belonging to the Response Theory to a given item in order to evaluate each one of the replies that the subject gives to each one of the questions that are posed one by one, in such a way that the result is an evaluation of the quality of life related to the health.

Also reference is made to the procedure developed for the assessment of the items and their possible replies that form a part of the system and for the formulation of the internal rules of the system used to determine the number and order of the items presented to the subject.

BACKGROUND TO THE INVENTION

The present invention concerns in the area of medicine that is concerned with the evaluation of results of the practical clinic (diagnostic and therapeutic) from the point of view of the patient that is the object of it and that is known as assessment of results in health. This discipline used the so called "measurements reported by the patient" of which the most used is the quality of life related to health (QLRH).

Said variable can be defined as the subjective evaluation of the influence of the state of health, the health care and the promotion of health over the ability of the individual to have a working level that allow them to follow those activities that are important and that affect their well being.

Currently the only way of evaluating the QLRH of a person is by means of the use of standardised questionnaires, designed from the assumptions of the Classical Theory of Tests and therefore formed by a fixed number of questions that once answered can be marked according to some fixed and equal rules for all subjects. Amongst the most internationally used classic QLRH questionnaires are the SF-36 and EQ-5D Health Questionnaires.

The SF-36 Health Questionnaire is a generic instrument for measuring the perceived state of health, applicable to any group of people, that allows comparisons to be established with the general healthy population and for which a self-assessment system has recently been developed which has been the object of an application for industrial property protection in Spain (N° 200600211).

From the psychometric point of view, it deals with a tough questionnaire, especially when dealing with the evaluation of groups of subjects, not so much for the individual patient, given that the reliability coefficient is located below the limit of 0.9 proposed as the ideal for questionnaires that one wishes to use in individual evaluations and follow ups.

According to the current state of the technique, this questionnaire, like other similar ones, can be completed in writing (the person answers some questions on paper), by personal or telephone interview (in both cases the interviewer asks some questions and writes down the answers given by the person being interviewed) and also, in the exclusive case of the SF-36 Health Questionnaire, by means of the system for which protection has been applied for (invention patent application N° 200600211), in approximately 10 minutes.

The evaluation QLRH in the sphere of chronic diseases is more and more taking on relevance. The main reasons to explain this change can be summed up in the following points:

The determination of the effectiveness of the medical intervention;
The improvement in the taking of clinical decisions;
The evaluation of the quality of the care;
The estimation of the needs of the population; and
The understanding of the causes and consequences of the differences in health.

For some authors, the evaluation of the QLRH by the patient is especially important in the assessment of the consequences of the chronic diseases, because the clinicians need information about the effects of a specific illness on their patients and also the effect of specific treatment, in order to be able to improve the management of the disease and to assess the evolution of the patient.

In addition, it has been clearly shown that the judgements of the clinicians and patients differ in a significant manner. Because of this, a systematic evaluation of the QLRH in the practical clinic would allow for the improvement of the medical criteria in the treatment of chronic patients.

On the other hand, in the last few years, the evaluation of the results of the medical interventions has tended to be focussed on the patient, data referring to the quality of life related to health expressed by the patient acquiring relevance, likewise the degree of satisfaction with the treatment and the preferences at the point of choosing between alternatives.

In the last phase of the development of new treatments, in addition to studying aspects related to effectiveness, safety and the forecast of costs of the new medicine, the pharmaceutical companies tend to include these aspects related to the opinion of the patient in regard to the treatment, that can be very relevant in the approval of the medicine, likewise in the determining of the sales price.

There are different disadvantages that prevent the use of these means of assessment in clinical practice and which have encouraged significant work intended to solve them, such as that which has brought about the stated patent N° 200600211.

Amongst these disadvantages are: 1) the difficulty of interpreting the marking given by the clinician in charge of the patient in each case, hence the intervention of experts in the evaluation of QLRH is usually necessary; 2) the lack of time that exists in normal clinical practice to deliver the evaluation questionnaire, explain to the patient how to fill it in; give time to the patient to fill the questionnaire in and finally collect it for the subsequent introduction of the answers into a database and correct it, for the intention of obtaining the marks that represent the view of the patient regarding the area of health that is being assessed; 3) the consumption of time and money involved in the entirety of the tasks needed to carry out the assessments: The printing out on paper of the questionnaires, time for the professional that carries out the interviews, for the one tabulating the data in the database and for the one who corrects the data tabulated in order to obtain the marks of the dimensions of the questionnaire. In addition, until the presentation of patent N°200600211 there has not been any specific procedure to assess the changes in the time of the patients, beyond the normally used statistical trials, which on the other hand, are also not known by the users outside of the specific groups of experts.

In spite of the fact that patent N° 200600211 solved some of the different existing disadvantages that prevented the use of these assessment measures in clinical practice, some problems remain without there being solutions for all of them arising from the limitations imposed by the Classical Theory of the Tests to the development of QLRH questionnaires: All of them have a high number of items (for example the 36 questions of the SF-36 Health Questionnaire) and the marking has an error that is too big for the individual estimations to be considered as reliable either in an isolated manner or over time in successive assessments.

The so called Computer Adaptive Testing are questionnaires developed using the principles and methods of the Response Theory to the Item (RTI), whose intrinsic characteristics convert them into tools capable of overcoming these problems. In some other countries some computerised systems have been developed for the dynamic assessment of the state of health (such as that contained in the patent N° US 2002019747) that assesses the patient using a reduced number of questions and, in spite of this with a very small margin of error. Both characteristics, in the area of evaluation of quality of life related to health, would allow the two previously mentioned hurdles to be dealt with.

Basically a computer adaptive test is a personal psychological assessment test that is used by means of a computer and whose distinctive characteristic is that the presentation of the items is carried out in line with the level, on a varying degree, that the person being evaluated is showing. The Response Theory to the Item provides a methodological framework with important advantages over the classical theory of the tests for the analysis of the psychometric properties of a measure of these characteristics.

## DESCRIPTION OF THE INVENTION

Starting out from that described previously, one aim of the present invention is to provide a procedure to evaluate the quality of life related to the health of a person, which is adapted to the degree in the measure expressed by the subject being evaluated, putting forward a reduced number of questions and making an assessment with great accuracy.

This aim is achieved according to claim 1, by means of a procedure to evaluate the quality of life related to the health of a person from the health data introduced by said person, according to a number of between 5 and 15 questions put forward, chosen by the system from those which make up a bank of items calibrated by Response Theory to the Item, in a computer system that encrypts said health data, said procedures having the following stages:

(a) The obtaining of the first datum: To obtain the coded or encrypted response to the first question put to the subject being assessed.
(a) The decoding of the first datum: To decode the response to the first question;
(c) Generation of parameters: To generate specific parameters associated to the answer given to the first question asked, from the decoded datum and by means of the calculation algorithm developed by the Response Theory to the Item; one of the parameters provides a provisional estimation of the mark for the quality of life related to the health of the subject and its corresponding typical error and the others, calculated according to the above and one for each item in the calibrated bank of items, they are used in the selection of the following question;
(d) Selection of the next question to be put to the subject being evaluated: The system selects a new question from amongst those that make up the calibrated bank of items using one of the parameters calculated in the previous stage;

(e) The obtaining of the following data: Successively repeating stages (a) to (d) while continuing to modify the estimation of the mark of the subject being evaluated until reaching one of the conditions for the termination of the procedure; and

(f) Evaluation of the quality of life related to the health: To determine the quality of life related to the health of the person, from the last parameter calculated.

In this way, a procedure is achieved to evaluate the quality of life related to the health of a person that requires an answer to very few questions (between 5 and 15) in a very short time and that in real time provides a very accurate estimation, for that specific person and in a specific moment in time.

In addition, the invention provides other advantages that are described below. In the first place it considerably reduces the work load that up to now was posed for the subject being evaluated which meant answering a number of questions that was almost never less than 30. The short number of questions that is put to the subject with the system here being presented and the fact that the questions are put one by one, allows the active incorporation of the patients themselves in the help process on considerably reducing the load that up to now was placed on the patient to answer classic questionnaires.

In second place, the fact that the questionnaire is answered on a computer system allows the time invested in the invention procedure to be considerably reduced: the health data introduced by the person can be tabulated directly in a database; the presence of technician is not necessary for the interview with the person, either physically present or by telephone, to deliver the questionnaire to the person, to explain how to fill it in, to collect it or to tabulate the data into the database or correct the health data introduced by the person. Said reduction of time is added to the short number of questions used to evaluate the subject with which the introduction of the invention into normal clinical practice is made easier.

On the other hand, the results obtained by the invention for the QLRH of the person are different to those obtained by the known systems. In no way could the known systems provide the results obtained by the invention. The results obtained with the system that is being presented here have a margin of error 3 times less than those obtained by the answering of classic questionnaires, in such a way that the follow up in the time of the individual patient, for example to evaluate the response to a treatment, is most likely to check the real changes that are being produced in the quality of life related to the health.

Clearly, the completing of the questionnaire can be carried out on any computer system, either with a personal computer, a laptop, a pc tablet or a PDA.

According to one characteristic of the invention, the procedure can include a health data tabulation stage (g), to structure and store the data on the quality of life related to health in a first storage place. As and how was stated previously, said tabulation is carried out automatically from the introduction of the health data by the person, and does not require the involvement of a technician.

The procedure can also include a parameter tabulation stage (h), to structure and store the parameter in a second storage place, said first storage place and said second storage place being able to be in the same place. In the same way, said tabulation is carried out automatically and, therefore, does not require the involvement of a technician.

According to a preferred embodiment of the invention, stage (f) of the evaluation of the quality of life related to health includes the sub-stages of:

(f.1) To obtain the result of the final parameter calculated from the response to the last question put to the subject;
(f.2) To interpret said result; and
(f.3) To show said interpretation of the result.

In this way, the procedure of the invention, in addition to allowing a direct interpretation of the results regarding the quality of life related to the health of a person, also provides the clinician with an interpretation of said results by means of a definition, in order to guide the clinician even more in the management of the patient.

The procedure can be repeated over time, and it can include a stage (i) for the comparison of results, to compare the results obtained in the sub-stage (f.1), over time, with which the invention can carry out a follow up of the person over time. The procedure can be carried out successively, in such a way that marks are obtained for the parameters calculated from the answers of the subject at several point in time, processing the successive health data introduced by the person with a statistical model that allows the obtaining of the qualification of the statistical meaning of the change that has occurred in the marking.

In the stage (a) of the obtaining of the first datum, the data can be obtained from a first computing system in which the health data are encrypted and are sent to a second computing system, at least one of the stages (a) to (i) being carried out in said second computing system. In this case, the first computing system and the second computing system can be connected to each other by means of a communication network, for example a global communication network, such as the Internet.

According to an embodiment of the invention, in stage (a) for the obtaining of the first datum, the first question put to the subject is chosen at random by the system from a reduced group of questions in the calibrated bank of items chosen for that purpose by the results of the previous analysis carried out applying the theory of the answer to the item to the statistical bank.

According to a preferred embodiment of the invention, in stage (c) of the generation of parameters the estimated value of the measurement of the quality of life related to health is calculated according to the replies of the subject to the questions posed, and also the function of information of each item in that provisional estimation is calculated. The following questions are chosen according to the criterion of maximum information: The item is chosen so that the information is maximum in the provisional estimation of the measurement obtained by the answer to the first question.

According to a preferred embodiment of the invention the selection process and the administration of items continues until one of two conditions is fulfilled: (a) that 15 items have already been administered or (b) that a minimum of 5 items has been administered, that the typical error of estimation is less than 1 and that the percentage of reduction of the typical error compared to its value after the immediately previous item is less than 5%. When the stop condition is fulfilled, the final estimation of the measurement is calculated with the same procedure (estimated value at a later stage) that had been used during the course of the trial in order to obtain the provisional estimations.

According to another aspect of the invention, a computer programme is supplied that includes programme instructions in order to make the computing system carry out the previously described procedure. Said computer programme can be stored in some storage means, such as some means of storage, a computer memory or a read only memory. On the other hand, the computer programme can be carried by an electrical carrier signal.

BRIEF DESCRIPTION OF THE DRAWINGS

For the better understanding of the invention that which has been stated a sheet of drawings is included in which an outline of a practical embodiment is made which is made by way of being an example and without being by way of limitation, in which:

Figure 1 shows an outline representation of the adaptive system in order to evaluate the quality of life related to the health of a person, according to the invention;
Figure 2 corresponds to the programme (3) of drawing 1 explaining the processes and calculations that includes;
In figure 3 the information functions of the selected items (excluding the first) are shown; and
In figure 4 the evolution of the function of probability is shown, the dark lines corresponding to the last items applied.

DESCRIPTION OF THE PREFERRED EMBODIMENTS

A description of a preferred embodiment of the invention will be made below in which a bank of previously calibrated items is used to evaluate the quality of life of a subject, the questions being presented one by one and chosen from those which make up the bank in which the information function, calculated after the response to the item answered by the subject, is the maximum possible.

These items were formulated from the questionnaires being used in our country, introducing a series of modifications in the drafting of the questions and the answers from which the subject can choose. In addition, new items were added that make reference to aspects of quality of life related to the health of a subject that were not suitably covered by the set of initially defined items. This bank of items was calibrated by means of different studies with subjects that answered them, in such a way that at the end of the process a function was constructed that gives a value to the information provided by each item depending on the level in the variable to be measured, assigning a value to each item with a theoretical scale from 0 to 1.

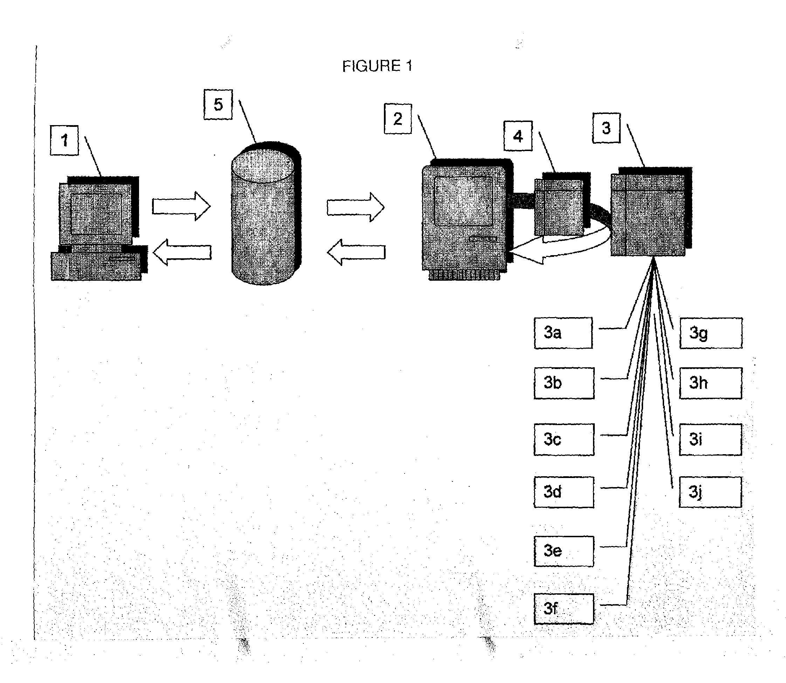

As can be seen in Fig 1., the adaptive system to evaluate the QLRH of a person or patient consists of a first computer 1 into which the patient introduces the response to the first question put which is chosen at random by the system from a reduced group of questions from the bank of calibrated items, chosen according to the result of previous analysis carried out applying response theory statistics of the item to the bank. The response is encrypted by said computer 1 and sent to a second computer 2. The second computer 2, which acts as a server, on which a programme in computer 3 is executed by means of which the estimated value is calculated for the measurement of the quality of life related to health, according to the response of the subject to the question asked, and also the information function of each one of the remaining items that form the bank 4 is calculated in that provisional estimation. The following questions are chosen from amongst those that make up the bank 4, according to the criterion of maximum information: The item is chosen so that the information is maximum in the provisional estimation of the measurement obtained by the answer to the first question. A local area network (LAN) 5, for example, the hospital network, connected to the first computer 1 and the second computer 2. In the case in which the first computer and the second computer are in different locations, for example, the first computer in the home of the patient and the second computer in the hospital, the communication network could be a global communication network, such as the Internet.

The process for the selection and administration of the items continues until one of two conditions is met: (a) that 15 items have already been administered or (b) that a minimum of 5 items has been administered, that the typical error of estimation is less than 1 and that the percentage of reduction of the typical error compared to its value after the immediately previous item is less than 5%. When the stop condition is fulfilled, the final estimation of the measurement is calculated with the same procedure (estimated value at a later stage) that had been used during the course of the trial in order to obtain the provisional estimations.

The first computer 1, which acts as client computer, has a computer programme that allows it to deal with the questions put to the subject, from amongst those that make up the bank of items, in an electronic format, which allows said health data obtained to be tabulated automatically in the database. In addition, it can encrypt the health data provided by the patient, in order to protect them from being accessed by non-authorised third parties, when they are stored in the first computer or when they are sent to the second computer 2 across the network. Said encryption can be carried out automatically or it can be at the wish of the patient or computer operator. Clearly, the first computer and the second computer can be the same computer, this means, the patient introduces the health data directly into the computer that determines and interprets the results on the quality of life related to the health of the patient, and hence the computer network 5 is not necessary.

Computer programme 3 has a module 3a in order to obtain the encrypted health data, introduced by the patient into the first computer 1 (said data can be sent by the first computer to the second computer or can be obtained by the second computer by means of access to the first computer); a module 3b in order to decode the health data; a module 3c to tabulate said health data in a database that has "a primary key", the identification of the patient; a module 3d to generate the marking from the health data of the patient; a module 3e to evaluate the information function of the remaining items in the bank 4; a module 3f to choose the next question to be put to the patient ; a module 3g in order to obtain the final mark of the QLRH of the subject or patient; a module 3h to interpret said results and to define the quality of life related to the health of the patient; a module 3i to show the results obtained, likewise the definition of the QLRH of the patient, for example, by means of a user graphical interface, in the first computer 1 or by means of any other computer terminal, in order to guide the clinician who is managing the treatment; and a module 3j to compare the results, which allows the evolution of the patient to be controlled over time.

The above stated database can be located in the second computer 2, although it could be in any other location. In addition, the health data and the final marks could be tabulated in different databases, each one of them being able to be in different locations.

Module 3d uses a data table that has been previously calculated by experimentation to generate the estimated marking of QLRH that includes the information that is necessary to estimate the probability that a subject with a specific level in the measured variable provides to each one of the possible answers of each item.

The coefficients used are the result of a complex statistical analysis carried out on experimental subjects.

In order to evaluate the information function of the remaining items from the bank module 3e carries out its calculation according to a previously designed statistical programme that is based on the Samejima graduated response model, that is commonly used in these types of processes.

Module 3f to select the following question automatically chooses the one which is the possible for the information function from those available (not presented to the subject or patient).

After successive repetitions of the module 3a to 3g operations module 3g assigns a final mark to the subject or patient, once the system has been stopped for one of the above described reasons.

Module 3h provides the interpretation of the mark assigned to the subject or patient who has answered the questions put by the system in such a way that it is standardised to a theoretical distribution of 50 plus a series of points using the following formula:

$$\text{Mark} = 50 + (\text{Final Mark} * 10)$$

Module 3i generates a graphical user interface and sends it to the first computer 1 in order to show the results obtained and likewise the definition of the quality of life related to the health of the patient, even though it could be shown on the screen of the second computer 2 itself or on the screen of any other computer terminal.

In order to compare the results module 3j obtains marks from the system over time, processing the successive answers from the patient or subject with the parametric statistical Module.

An example is described below that attempts to show the application of the invention, in a real situation, of a person with quality of life related to very deteriorated state of health.

The subject fills in the answers that the system puts forward in the first computer 1 that is connected to the network 5 of the hospital, in the framework of clinical follow up that is being carried out by the corresponding physician in regard to the chronic pathology.

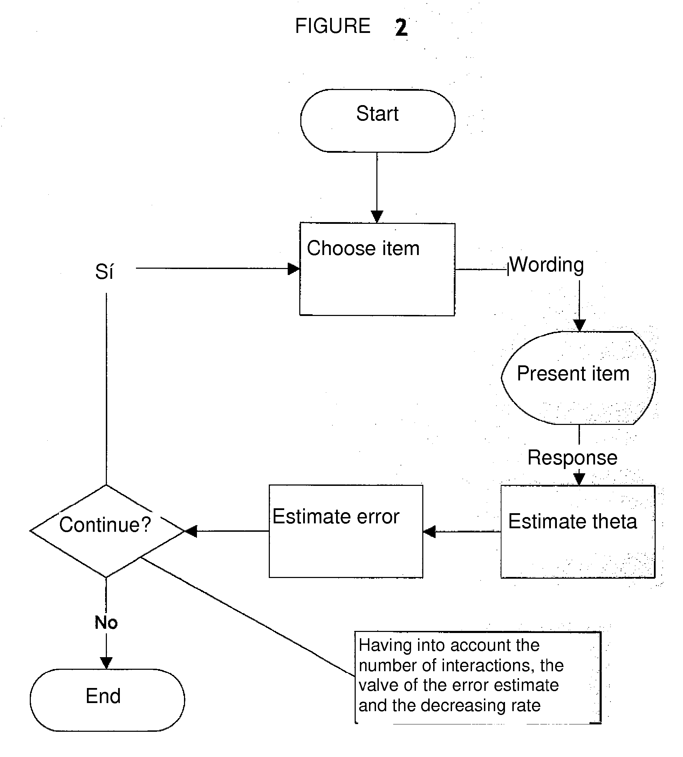

As and how described previously, the responses of the patient are sent to the second computer 2, in which after receiving the reply to the last proposed item, the values are calculated that take the function of probability in each point of the plausible domain of the variable to be measured ($\theta$) (See fig.2). The point that goes on to have the greatest probability will be the new value of theta.

Once the new estimation of theta has been obtained, the information functions of the items are examined that have not yet been applied, and the one that gives most information in the current situation is taken. The responses are tabulated on the basis of the corresponding data.

The questions chosen by the system in the example that is being presented here would be those that are presented below together with the estimated value of the variable to be measured ($\theta$) alter each reply, the estimated error and the % error reduction.

| Heading | Response | Estimated Theta | Estimated error | Error Reduction (%) |
|---|---|---|---|---|
| Do you think that things will get worse in the future | In complete agreement | -2,33 | 1,03 | |
| Do you have difficulty moving your feet | A lot | -3,25 | 1,55 | - 0,51 |
| Because of your health do you have difficulty bathing or showering | A lot | -3,63 | 1,53 | 0,01 |
| Do you need help to lie down | Always | -3,68 | 1,39 | 0,10 |
| Do you need help when you go to the toilet | Always | -3,72 | 1,20 | 0,13 |
| Does the state of your health stop you making your own meals | Always | -3,76 | 1,02 | 0,15 |
| Do you need help to get out of bed | Always | -3,81 | 0,94 | 0,07 |
| Does your health make it difficult to go out visiting | Always | -3,82 | 0,85 | 0,09 |
| Do you feel worn out | Always | -3,84 | 0,75 | 0,13 |
| Do you fall over easily | In complete agreement | -3,85 | 0,73 | 0,02 |

As can be seen in the above table after 7 items, the value of error already was coming within that which is acceptable, but it was still being reduced with each item, in such a way that it continued until the estimated error was stabilised.

Now as an example using the replies of a person with a very good quality of life related to health:

| Heading | Response | Estimated Theta | Estimated error | Error Reduction (%) |
|---|---|---|---|---|
| Do you feel full of energy | Often | 1,53 | 2,00 | |
| Because of your physical health do you do less things than you would like to do | Complete disagreement | 2,01 | 2,41 | - 0,20 |
| Your health is | Good | 1,82 | 1,23 | 0,49 |
| Because of feeling sad or agitated do you spend less time on your daily activities | Complete disagreement | 2,03 | 1,14 | 0,07 |
| Do you feel depressed | Almost never | 1,87 | 0,96 | 0,16 |

(continued)

| Heading | Response | Estimated Theta | Estimated error | Error Reduction (%) |
|---|---|---|---|---|
| Do you feel bad | Almost never | 1,92 | 0,96 | - 0,01 |
| Your health is good | In complete agreement | 2,03 | 1,03 | - 0,07 |
| Do you feel sad | Almost never | 1,99 | 0,82 | 0,20 |
| Do you feel down | Almost never | 1,98 | 0,80 | 0,03 |

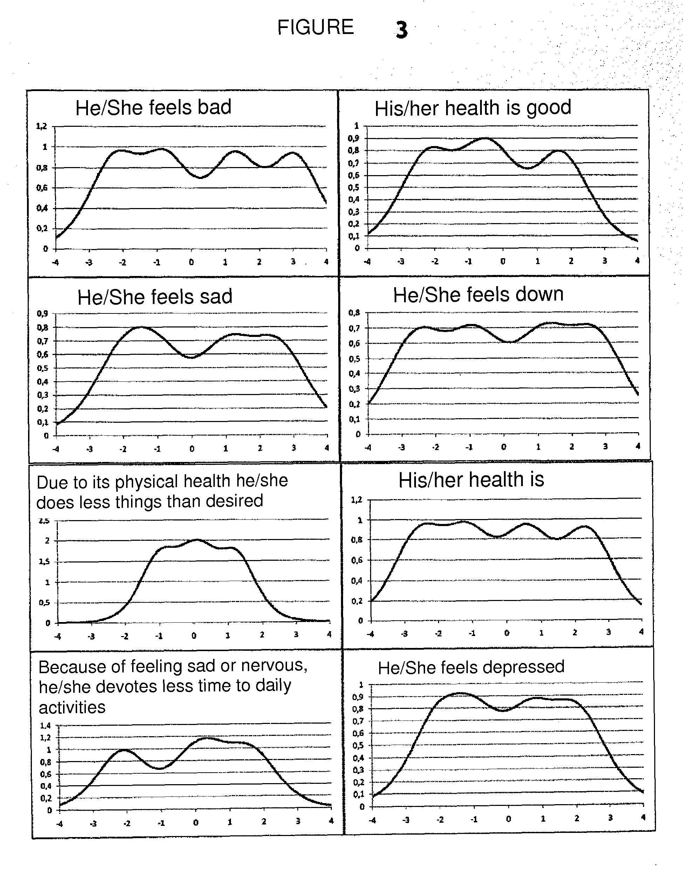

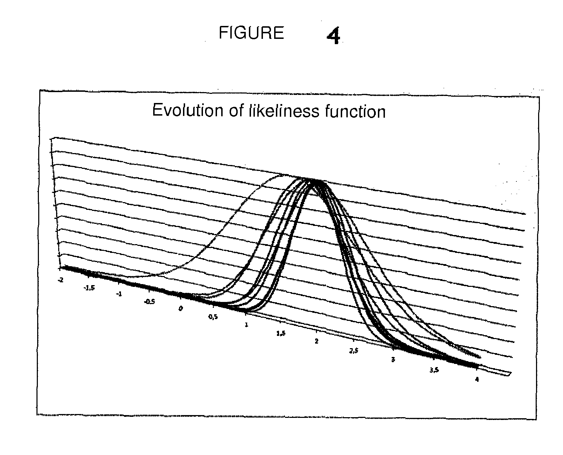

This last example will be analysed in somewhat more detail. If we observe the information functions of each one of the items selected (Fig. 3) we can see that as each item provides quite a lot of information around the value of the estimation of theta that brought about its selection (the first item around 1.5, the others around 2). In the evolution of the function of probability (Fig. 4) we can see the estimation is more and more exact (the curve is more and more sharp).

In spite of the fact that specific embodiments of this present invention have been described and represented, it is clear that the expert in the matter will be able to introduce variations and modifications, or replace details for others that are technically equivalent, without moving away from the sphere of the protection defined by the attached claims.

Also in spite of the fact that the embodiments of the inventions described with regard to the drawings include computing systems and processes carried out in computing systems, the invention is also extended to computer programmes, more specifically to computer programmes in or on carrier devices, adapted to put the invention into practice.

The computer programme can be in the form of source code, object code or in intermediate code between source code and object code, such as in a partially compiled form, or in any other suitable form to use in the implementation of the processes according to the invention. The carrier medium can be any entity or device capable of carrying the programme.

For example, the carrier means can include a means of storage, such as a ROM, for example a CD ROM or a semiconductor ROM, or a means of magnetic recording, for example a floppy disc or a hard disc. In addition the carried device can be a transmittable carrier device such as an electrical or optical signal that can be transmitted via electrical or optical cable or by means of radio or some other means.

When the computer programme is contained in a signal that can be transmitted directly via cable or some other device or means, the carrier means can be made by said cable or other device or means.

Alternatively, the carrier device can be an integrated circuit into which the computer programme is embedded, said integrated circuit being adapted to carry out, or to be used in the carrying out of the relevant processes.

**Claims**

1. Procedure to evaluate the quality of life related to the health of a person from the health data introduced by said person, responding to successive questions that the system poses according to the parameters that are being calculated at each reply, in a computer system (1; 2) that encrypts said health data, said procedure including the following stages:

   (a) Obtaining of the first datum in which the encrypted reply is obtained from the first question put to the subject being evaluated;
   (b) The decoding of the data: To decode the response to the first question;
   (c) Generation of the parameters in which specific parameters are generated that are associated to the answer given to the first question put, from the decoded datum and by means of the calculation algorithm developed by the Response Theory to the Item;
   (d) Selection of the next question to be put to the subject being evaluated in which the system selects a new question from amongst those that form the bank of items calibrated using one of the parameters calculated in the previous stage;
   (e) Obtaining the following data, successively repeating stages (a) to (d) while continuing to modify the estimation of the mark of the subject being evaluated until reaching one of the conditions for the termination of the procedure; and
   (f) Evaluation of the quality of life related to health in which the quality of life related to the health of a person is determined from the last parameter calculated.

2. Procedure according to Claim 1 **characterised in that** it includes a health data tabulation stage (g), in order to structure and store the health data in a first location.

3. Procedure according to claims 1 or 2 **characterised in that** it includes a parameter tabulation stage (h), to structure and store the parameter in a second location.

4. Procedure according to any of claims 2 or 3 **characterised in that** the first and second locations are a single location.

5. Procedure according to any of claims 1 to 4 **characterised in that** the evaluation stage (f) of the quality of life related to health includes the sub-stages of:

   (e.1) To interpret said result of the health questionnaire; and
   (e.2) To show said interpretation of the result.

6. Procedure according to any of claims 1 to 5 **characterised in that** it is repeated over time, and **in that** it includes a results comparison stage (i), in order to compare the results obtained in stage (f) over time.

7. Procedure according to any of claims 1 to 6 **characterised in that** the data obtaining stage (a), the data obtained from a first computer system (1) in which the health data are encrypted and are sent to a second computer system (2), and **in that** at least one of the stages (a) to (i) are carried out in the said second computer system.

FIGURE 1

FIGURE **2**

FIGURE **3**

He/She feels bad

His/her health is good

He/She feels sad

He/She feels down

Due to its physical health he/she does less things than desired

His/her health is

Because of feeling sad or nervous, he/she devotes less time to daily activities

He/She feels depressed

FIGURE **4**

Evolution of likeliness function

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 200600211 A
- US 2002019747 A